# EUROPEAN PATENT APPLICATION

(11) **EP 1 402 859 A1**
(43) Date of publication of application: **31.03.2004**
(21) Application number: 03380097.0
(22) Date of filing: 16.04.2003
(51) Int. Cl.: A61F 9/007

(54) **Tubular prothesis for draining the tear from the eye to the nasal fossa**

(30) Priority: 10.07.2002 ES 200201616
(71) Applicant: Murube del Castillo, Juan, 28034 Madrid (ES)
(72) Inventor: Murube del Castillo, Juan, 28034 Madrid (ES)
(74) Representative: Manzano Cantos, Gregorio

(57) **Abstract**

A tubular prosthesis for draining the tear from the eye to the nasal fossa, constituted of a single piece in light, malleable, and resistant biocompatible plastic materials and formed by a tube with fine outer and inner diameters and a length suitable for being placed between the lacrimal of the eye and the nasal vestibule, and which comprises an upper enlargement for its proper placement in the rima palpebrarum and another or other intermediate enlargements in order to remain secured by the post-surgical scar.

## Description

The invention consists of a tubular prosthesis of suitable length and a very fine diameter designed to be implanted between the rima palpebrarum and the nasal fossa through the soft parts of the face, which permits draining the tear from the eye to the nasal fossa, such that the surgical placement of this prosthesis does not require perforating any bone of the face and is carried out by means of a specific process created by the inventor itself and which has been developed and disclosed in another patent of the same inventor.

As is well known, the natural lacrimal pathways drain the tear from the eye to the nasal fossa. When these pathways are obstructed and it is not possible to surgically make them permeable again, a permanent prosthesis can be placed which passes over the obstructed area, a prosthesis which logically has the optimum conditions for its implantation, for its functioning and for preventing the rejection thereof.

The prosthesis in this case proposed by the invention is an improved tubular prosthesis having the particularity of being manufactured integrally of plastic, preferably of a resilient, malleable and resistant medical grade silicone and in a single piece with the means necessary for collecting the tear and carrying it through the tube until the nasal cavity, preventing the tube from shifting downwards and other means for preventing the tube from shifting upwards or downwards, which will be secured in the post-surgical scar.

### BACKGROUND OF THE INVENTION

Attempts to use tubular prostheses following the natural lacrimal pathways have been numerous, but none of them have lasted in time.

More successful was the "Jones" prosthesis, which joins both structures mentioned (oculoconjunctival sac and nasal fossa), by a pathway different from the natural one, with a tube made of Pyrex® glass, being of 1 to 2 cm. in length, crossing over the ascending ramus of the jaw bone and which is horizontally placed between the internal angle of the eye and the nasal fossa, which hinders flow through the tube.

This solution appeared almost forty years ago and none of the published modifications has surpassed the "Jones" tube, thereby not lasting, such that the "Jones" tube is still the only current surgical possibility.

Among the drawbacks of said system are the drawback that it requires a laborious surgical intervention, with perforation of the ascending ramus of the upper jaw bone, and the fact that its trajectory is almost horizontal, which does not facilitate flow through the tube.

Likewise, the prosthesis and solution proposed by the invention has been experimented on for over twenty years, the results of the first prototypes were published in 1982 (Murube del Castillo J. Conjunctivorhinostomy without osteal perforation. Arch. Ophthamol. 1982; 100: 310-311), and it is currently thought to be sufficiently improved so as to grant its registration.

A preceding version by the same inventor with which the purposes of the invention are achieved and which also discloses the surgical procedure for its placement in front of the upper jaw bone and under the soft parts of the face, is included in P. 200100251 as: "PROTESIS PARA DRENAR LA LAGRIMA DEL OJO A LA FOSA NASAL" (PROSTHESIS FOR DRAINING THE TEAR FROM THE EYE TO THE NASAL FOSSA), in which the prosthesis contained therein is a prosthetic tube susceptible of being manufactured of glass or plastic, provided with a nozzle assembled on the upper end of the tube, which can also be glass or plastic, a nozzle being a part suitably adapted to the tube.

### INVENTIVE STEP

The idea of the invention is that the entire prosthesis be formed by a single piece which, including the essential features of the former one, is integrally manufactured of plastic or a biocompatible synthetic material with the texture and ductility recommended to adapt internally to the palpebrarum and osteofacial anatomy, with a very fine section and long enough so as to reach the nasal cavity from the lacrimal.

### DESCRIPTION OF THE INVENTION

According to the features of the invention which is being disclosed, the prosthesis is significantly constituted by a tubular element or part manufactured of plastic material, preferably a medical grade silicone, with a fine outer diameter and smaller inner diameter or bore and with a length comprising at least the distance between the lacrimal and the nasal vestibule, preferably with a relative longer length in order to cut or remove the excess part, perfectly adapting it to the necessary measurement.

A tube according to the invention having an outside enlargement or rim on its upper end, on the peripherally encircled opening, widened said end for perfect palpebrarum adaptation thereof against the angular internal anatomy of said lacrimal, placed for directly collecting the secretion which is carried through the tube to the nasal fossa such that the rim prevents the prosthesis from shifting downwards, being displaced from its proper palpebrarum location.

Likewise, another feature of said tube according to the invention is that it comprises several, preferably made up of three, intermediate projections in toric or casing position and in preferably rectangular-shaped format with eased edges, which remains secured in the post-surgical scar, preventing the prosthesis from shifting upwards or downwards.

Another characteristic detail of the invention is that said tube has a preferred length between 6-8 mm, more specifically of 7.905 mm, and an outer diameter of 2 mm and inner diameter of 1.5 mm, and more specifically an outer diameter of 1.96 mm and an inner diameter of 1.4 mm.

A broader idea of the features of the invention will be carried out below in reference to the sheets of drawings attached to this specification in which, schematically and only as an example, the preferred details of the invention are shown.

### IN THE DRAWINGS

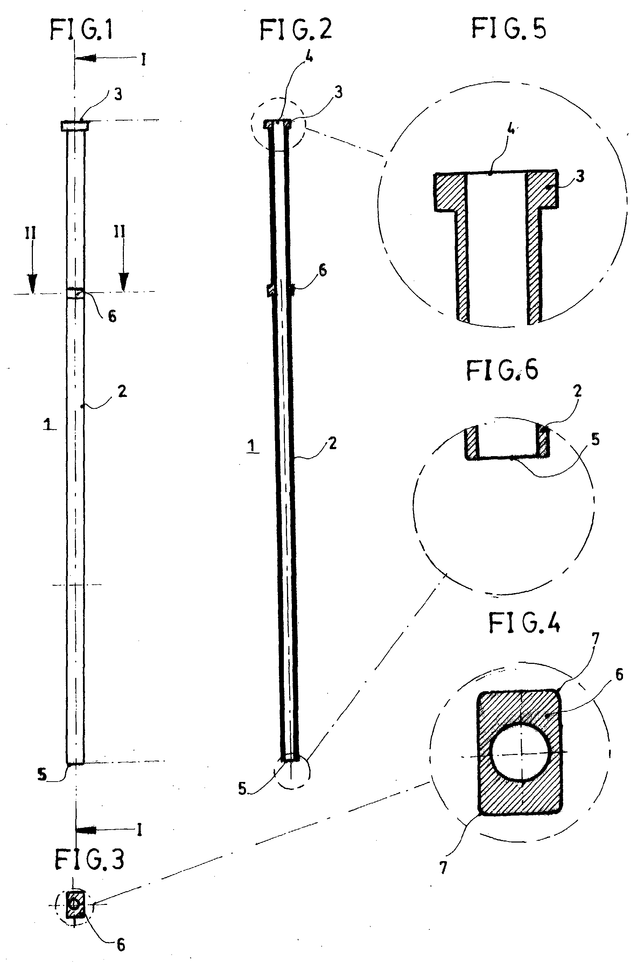

Figure 1 shows a front elevational view of the entire prosthesis.
Figure 2 shows a view, longitudinally sectioned along line I-I of figure 1.
Figure 3 shows a view, transversally sectioned along line II-II of figure 1.
Figure 4 shows an enlarged detail of figure 3.
Figure 5 shows an enlarged detail of the upper end of figure 2.
Figure 6 shows an enlarged detail of the lower end of figure 2.

### PREFERRED EMBODIMENT OF THE INVENTION

Said drawings describe a prosthesis according to the interpretation of the invention in a single piece (1), integrally manufactured from plastic or a synthetic material, more specifically a biocompatible plastic, such as light, malleable and resistant medical grade silicone, formed by a tube (2) of 1.96 mm in outer diameter and 1.47 mm in inner diameter and 8 mm in length, in order to be placed between the lacrimal of the eye and the nasal vestibule, cutting or removing the excess part as needed.

Said tube (2) has an upper enlargement (3) formed by an outer rim around the upper opening (4) permitting its adaptation to the rima palpebrarum and preventing its downward sliding and being useful to collect the tear from the inner angle of the eye, carrying it through the tube so that it comes out through the lower end (5); furthermore being provided with one or more, preferably intermediate, toric enlargements of a preferably rectangular shape with rounded edges (7), such that, secured by the post-surgical scar, they prevent the tube from shifting upwards and downwards.

Having suitably disclosed the nature of the invention, it is hereby stated to the right effect that the invention is not limited to the exact details of this description, but rather on the contrary, those modifications deemed suitable will be introduced in the invention as long as the essential features thereof, which are claimed below, are not altered.

## Claims

1. A tubular prosthesis for draining the tear from the eye to the nasal fossa, which is implanted according to the suitable surgical procedure and which is **characterized by** being integrally formed by a single piece (1) of a light, malleable, and resistant biocompatible plastic material which has a tube (2) with fine inner and outer diameters and of a length suitable for being placed between the lacrimal of the eye and the nasal vestibule and which comprises an upper enlargement (3) and another or other toric and preferably tilted intermediate enlargements (6).

2. A tubular prosthesis for draining the tear from the eye to the nasal fossa according to claim 1, the material of the tube (2) is **characterized in that** it is preferably medical grade silicone.

3. A tubular prosthesis for draining the tear from the eye to the nasal fossa according to claim 1, the diameters of said tube (2) are **characterized in that** the outer diameter is approximately 2 mm, and more specifically 1.96 mm, and the inner diameter is approximately 1.50 mm, and more specifically 1.47 mm.

4. A tubular prosthesis for draining the tear from the eye to the nasal fossa according to claim 1, the length of the tube (2) is **characterized in that** it will preferably be 8 mm.

5. A tubular prosthesis for draining the tear from the eye to the nasal fossa according to claim 1, the upper enlargement (3) is **characterized in that** it is an outer rim encircling the upper opening (4) for collecting the tear from the inner angle of the eye and preventing the tube from slipping downwards.

6. A tubular prosthesis for draining the tear from the eye to the nasal fossa according to claim 1, the intermediate toric enlargement or enlargements (6) are **characterized in that** they are preferably rectangular (6) in shape with rounded edges (7) which prevent the tube (2) from slipping upwards and downwards when secured by the post-surgical scar.
